# EUROPEAN PATENT APPLICATION

(11) **EP 1 749 477 A1**
(43) Date of publication of application: **07.02.2007**
(21) Application number: 05736958.9
(22) Date of filing: 28.04.2005
(51) Int. Cl.: A61B 5/18, A61B 5/0245, A61B 5/0452, B60K 28/06

(54) **BIOINFORMATION SENSOR**

(30) Priority: 24.05.2004 JP 2004153421
(71) Applicant: Pioneer Corporation, Tokyo 153-8654 (JP)
(72) Inventor: YANAGIDAIRA, M. Pioneer Corporation, gashima-shi Saitama (JP); YASUSHI, Mitsuo Pioneer Corporation, gashima-shi Saitama (JP); SHIODA, Takehiko Pioneer Corporation, gashima-shi Saitama (JP)
(74) Representative: Haley, Stephen
(86) International application number: PCT/JP2005/008251
(87) International publication number: WO 2005/112764

(57) **Abstract**

In a bio-information detecting apparatus, whether detection of heartbeats by a steering wheel sensor is possible is judged. When it is judged that the detection by the steering wheel sensor is impossible, whether detection of heartbeats by an installed-in-seat sensor is possible is judged. In this judgment, a judgment based on an output from an acceleration sensor and a judgment based on a heart-rate fluctuation range obtained from an output from the steering wheel sensor are executed.

## Description

### TECHNICAL FIELD

The present invention relates to a bio-information detecting apparatus.

### BACKGROUND ART

The state of a vehicle driver is monitored and the information obtained from the monitoring is utilized for preventing the driver from dozing off at the wheel, releasing irritation of the driver, etc. As a method of monitoring the state of a driver, a method of detecting bio-information of the driver and grasping the state of the driver using this bio-information can be listed. For example, a method of detecting electro-cardio information as the bio-information can be listed. According to this method, appropriate monitoring that reflects more accurately the mental and physical states of the driver is enabled because the electro-cardio information reflects the action of the autonomic nerve of the driver.

Conventionally, as one of the methods of detecting electro-cardio information, a method can be listed, according to which means for detecting electro-cardio information are installed on a steering wheel for steering a vehicle (refer to, for example, Patent Document 1 and Patent Document 2). According to this method, for example, an electrode (hereinafter, "steering wheel sensor") is installed on an area that is grabbed by the right and left hands and the electro-cardio information is obtained by detecting the heartbeats of the driver from the potential difference between both hands detected by the steering wheel sensor.

As another method of detecting electro-cardio information, a method can be listed, according to which a pressure sensor is installed in an area such as a portion of the seat in the car under the buttocks, under the thighs, on the back, etc., of the driver (refer to, for example, Patent Document 3). According to this method, the electro-cardio information is obtained by detecting the heartbeats of the driver by detecting a vibration on the body surface associated with the heartbeats using the pressure sensors.

Patent Document 1: Japanese Patent Laid-Open Publication No. H6-255518
Patent Document 2: Japanese Patent Laid-Open Publication No. H10-146321
Patent Document 3: Japanese Patent Laid-open Publication No. H9-308614

### DISCLOSURE OF INVENTION

### PROBLEM TO BE SOLVED BY THE INVENTION

The method of detecting electro-cardio information using a steering wheel sensor has high precision and can obtain much information. However, the detection can not be executed unless the steering wheel is held with both hands, and the information can not be detected during one-handed steering. Therefore, the driver is forced to hold the steering wheel with both hands for the information to be detected and the driver is caused to notice the detection. Therefore, this may increase the load on the driver and interfere his/her steering.

According to the method of detecting electro-cardio information from a pressure sensor, the detection can be executed even when the driver does not notice the detection while the driver remains in a natural sitting posture thereof. However, the pressure sensor tends to receive unnecessary signals other than the heartbeats, which are mixed with the heartbeats and caused by the vibration associated with the behavior of the vehicle and move of the body of the driver, etc., while the vehicle is running. Therefore, the precision of the detection of the electro-cardio information is degraded.

### MEANS FOR SOLVING PROBLEM

A bio-information detecting apparatus according to the invention of claim 1 is installed in a vehicle, a ship, or an airplane to be steered and detects at least mental and physical states of a driver. The bio-information detecting apparatus comprises: an electrode sensor that includes at least a pair of electrodes and detects heartbeats by detecting potential difference between portions of a body that directly contact the electrodes; and an installed-in-seat sensor that detects heartbeats by detecting vibration of a surface of the body associated with the heartbeats, wherein the bio-information detecting apparatus is configured to be able to utilize heartbeat information obtained by detection by the electrode sensor for detecting heartbeats by the installed-in-seat sensor, and to judge whether detection by the installed-in-seat sensor can be executed based on the heartbeat information obtained by the electrode sensor when heartbeat detection by the electrode sensor can not be executed.

### BRIEF DESCRIPTION OF DRAWINGS

Fig. 1 is a schematic of a configuration of a bio-information detecting apparatus according to a first example of the present invention;
Fig. 2 is a flowchart of a drowsiness detecting method in the bio-information detecting apparatus shown in Fig. 1;
Fig. 3 is a schematic for explaining a method of detecting the degree of advancement of drowsiness based on fluctuation of a heart rate in the drowsiness detecting method shown in Fig. 2;
Fig. 4 is a schematic for illustrating detecting operation according to a second example of the present invention in which the drowsiness detecting method is applied to actual driving;
Fig. 5 is a flowchart of a drowsiness detecting method according to a third example of the present invention;
Fig. 6 is a schematic for explaining a method of creating a pressure-sensor output-template waveform in the drowsiness detecting method shown in Fig. 5;
Fig. 7 is a schematic for explaining a method of creating a pressure-sensor output-template waveform in the drowsiness detecting method shown in Fig. 5;
Fig. 8 is a schematic for explaining a method of creating a pressure-sensor output-template waveform in the drowsiness detecting method shown in Fig. 5;
Fig. 9 is a schematic for illustrating a method of creating the pressure-sensor output-template waveform and a created pressure-sensor output-template waveform in the drowsiness detecting method shown in Fig. 5;
Fig. 10 is a schematic for explaining detecting operation that uses the pressure-sensor output-template waveform in the drowsiness detecting method shown in Fig. 5;
Fig. 11 is a schematic for explaining detecting operation that uses the pressure-sensor output-template waveform in the drowsiness detecting method shown in Fig. 5;
Fig. 12 is a schematic for explaining detecting operation that uses the pressure-sensor output-template waveform in the drowsiness detecting method shown in Fig. 5;
Fig. 13 is a schematic of a configuration of a bio-information detecting apparatus according to a fourth example of the present invention;
Fig. 14 is a schematic of a configuration of a seat in a case when an angular velocity sensor or a rotation sensor are applied; and
Fig. 15 is a flowchart of a process of calculating a breath frequency using a pair of electrodes in a steering wheel unit.

### EXPLANATIONS OF LETTERS OR NUMERALS

- 100: STEERING WHEEL SENSOR
- 100a, 100b: ELECTRODE
- 100c: STEERING WHEEL MAIN BODY
- 110, 1301 to 1303: INSTALLED-IN-SEAT PRESSURE SENSOR
- 120: CONTROLLER
- 121: MEMORY UNIT
- 122: CALCULATING UNIT
- 123: JUDGING UNIT
- 124: CONTROL UNIT
- 125: INPUT SETTING UNIT
- 130: ACCELERATION SENSOR
- 140: REPORTING DEVICE
- 1304: CAR AUDIO SET
- 1305: SEAT ADJUSTING DEVICE

### BEST MODE(S) FOR CARRYING OUT THE INVENTION

One of the objectives of the present invention is to provide, for example, a bio-information detecting apparatus capable of executing high precision bio-information detection that does not cause a driver to notice the detection. Exemplary embodiments of the bio-information detecting apparatus according to the present invention will be described below.

### (EMBODIMENTS)

The bio-information detecting apparatus according to an embodiment of the present invention is mounted on a vehicle, a ship, or an aircraft that is a target to steer, and is a bio-information detecting apparatus that detects at least the mental and physical states of the driver thereof. This bio-information detecting apparatus includes an electrode sensor that includes at least a pair of electrodes and detects heartbeats by detecting the potential difference between portions of the body that directly contact the electrodes, and an installed-in-seat sensor that detects heartbeats by detecting the vibration of the body surface associated with the heartbeats. This bio-information detecting apparatus is configured to be able to utilize heartbeat information obtained by the detection by the electrode sensor during the heartbeat detection by the installed-in-seat sensor and, when the heartbeat detection by the electrode sensor can not be executed, whether or not the detection by the installed-in-seat sensor can be executed is judged.

For example, a steering wheel sensor constituted by a pair of electrodes respectively installed separately on the steering wheel installed at the seat of the driver, is used as the electrode sensor, and the pressure sensor for being installed in the seat, that are installed in a position corresponding to a portion of the seat under the buttocks, under the thighs, or on the back is used as the installed-in-seat sensor.

It is preferable that the bio-information detecting apparatus further includes a target-to-drive behavior sensor that detects behavior of a target to drive, and can utilize behavior information of the target to drive obtained by the detection by the target-to-drive behavior sensor during the heartbeat detection by the installed-in-seat sensor. In this case, when the heartbeat detection by the electrode sensor can not be executed, whether or not the detection by the installed-in-seat sensor can be executed is judged based on the behavior information obtained by the target-to-drive behavior sensor.

Furthermore, it is preferable that the bio-information detecting apparatus includes reporting unit that reports at least the driver about the bio-information obtained by the detection by the electrode sensor and the installed-in-seat sensor.

### First Embodiment

An example of a bio-information detecting apparatus according to the present invention will be described in detail below with reference to the accompanying drawings. In the example, a bio-information detecting apparatus to be installed in a car which detects bio-information concerning the mental and physical states of the driver based on electro-cardio information is exemplified and, especially, an apparatus that copes with detection of drowsiness of the driver is described.

Specifically, the heart rate and heartbeat fluctuation are detected as the electro-cardio information used for detecting the drowsiness of the driver. Though heartbeats are varied depending on the posture of the driver, the air temperature, the mental state of the driver, etc., the posture of the driver and the ambient temperature are not varied so often while driving. Therefore, the mental state of the driver such as drowsiness is the main cause that varies his/her heartbeats. The heartbeat fluctuation is a fluctuating component of the temporal interval of the beats of his/her heart (heartbeats). Usually, while the driver is not aware of his/her drowsiness, the value of HF described below does not fluctuate. However, when the driver feels drowsiness, the HF increases corresponding to the strength of the drowsiness.

Therefore, detecting the occurrence of drowsiness is enabled by detecting this fluctuation of the temporal interval of the heartbeats. Especially, in this example, a specific high frequency component (frequency 0.15 to 0.4 Hz) of the heartbeat fluctuation is referred to as HF (High Frequency) and the detection is executed using this component. As described later, in the drowsiness detection using the heart rate and the HF, advancement of the drowsiness from the state where the driver is not aware of his/her drowsiness can be grasped based on the amount of reduction of the heart rate, and the occurrence of the drowsiness can be grasped based on the increase of the HF.

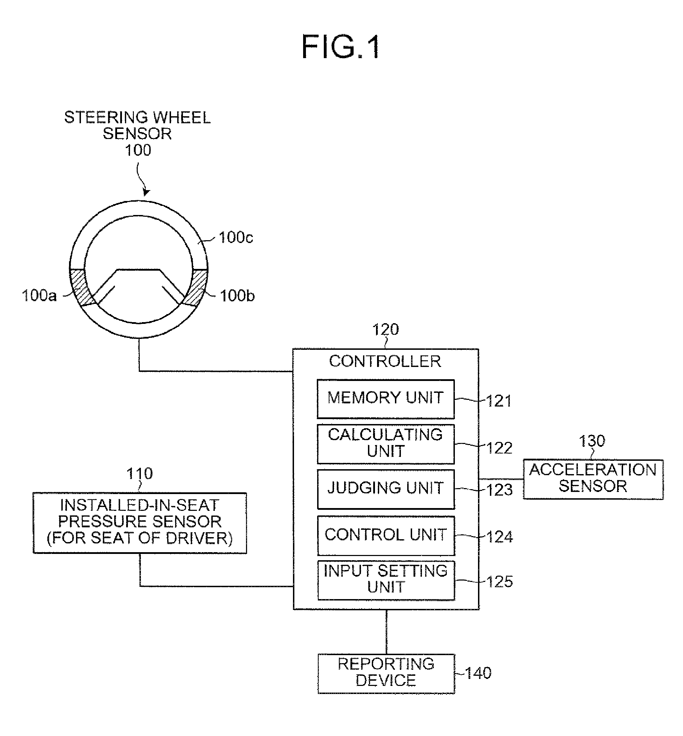

Fig. 1 is a schematic of an example of the configuration of the bio-information detecting apparatus according to a first embodiment of the present invention.

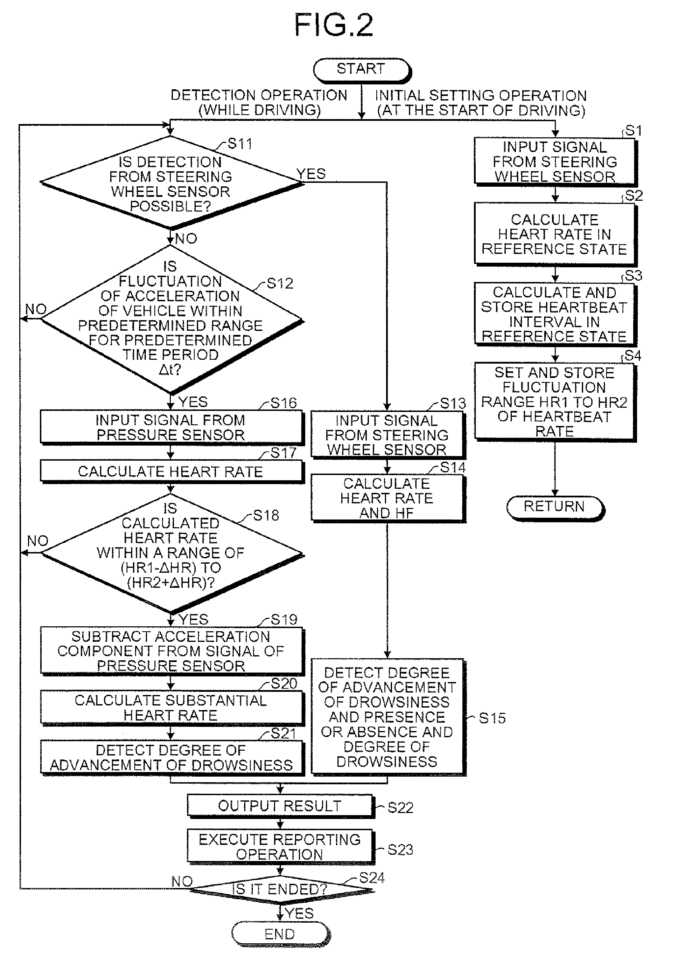

Fig. 2 is a flowchart of an example of a drowsiness detecting method in the bio-information detecting apparatus shown in Fig. 1.

As shown in Fig. 1, the bio-information detecting apparatus includes a steering wheel sensor 100, an installed-in-seat pressure sensor 110, an acceleration sensor 130 that is a vehicle behavior sensor, a controller 120, and a reporting device 140 as main components. The steering wheel sensor 100 includes a pair of electrodes 100a, 100b installed separated from each other to a ringshaped steering-wheel main-body 100c installed in a car. The steering wheel sensor 100 is configured such that the left hand contacts to the electrode 100a on one hand and the right hand contacts the electrode 100b on the other hand while a driver is operating the steering wheel.

The steering wheel sensor 100 can directly detect the potential difference between the hands obtained when the hands of the driver contact the electrodes 100a and 100b, as an electric signal as electro-cardio information (more specifically, electro-cardiogram) of the driver. Therefore, the heart rate and the HF can be detected from the electro-cardiogram obtained by the steering wheel sensor 100, and drowsiness can be detected.

As described later, in the detection by the steering wheel sensor 100, the electro-cardio information can be directly detected. Therefore, higher-precision detection is enabled compared to the detection by an installed-in-seat pressure sensor 110. Because not only the heart rate but also the HF can be detected, the amount of information collected is much and, therefore, especially, absolute evaluation as to presence or absence of drowsiness based on the HF is enabled. Therefore, the degree of advancement of the drowsiness can be detected based on the fluctuation of the heart rate, and presence or absence of drowsiness and the degree of the drowsiness can be detected using the HF. Therefore, both of relative detection and absolute detection of the drowsiness can be executed.

The installed-in-seat pressure sensor 110 is installed in a portion of the seat corresponding to a portion under the buttocks, under the thighs, or on the back of the driver when the driver sits on the seat of the driver (not shown) installed in the car in a natural posture. The position of the installed-in-seat pressure sensor 110 is not limited especially when the position is in a portion of the seat that is pressed by the surface of the body of the driver when the driver sits in the seat in a natural posture. For example, the plural installed-in-seat pressure sensors 110 may be installed in different positions of the seat.

As the installed-in-seat pressure sensor 110, a pressure sensor that can detect slight pressure variation such as a piezoelectric sensor formed as a thin film, a sensor configured to detect the variation of the air pressure, etc., can be used. In this example, a piezoelectric sensor is used as the installed-in-seat pressure sensor 110, and is configured to output an electric signal corresponding to the magnitude of the pressure received from the surface of the body of the driver, to the controller 120. The vibration of the surface of the body of the driver synchronized with the heartbeats is detected by the installed-in-seat pressure sensor 110 as the pressure fluctuation. As a result, the vibration of the surface of the body is detected as heartbeats, and the heart rate is calculated from the obtained heartbeats.

In this detection by the installed-in-seat pressure sensor 110, the detection is enabled when the driver only sits in the seat (not shown). Therefore, detection can be executed without being noticed by the driver. In the detection by the installed-in-seat pressure sensor 110, as described in the above "BACKGROUND ART" section, vibrations other than the heartbeats associated with the behavior of the car are detected. Therefore, the detected heartbeats include noise components other than the heartbeats. Because these noise components degrade the detection precision, in the bio-information detecting apparatus in this example, a process to facilitate improvement of the detection precision of the installed-in-seat pressure sensor 110 is executed as described later with reference to Fig. 2.

The acceleration sensor 130 detects the behavior of the car from the acceleration thereof. This acceleration sensor 130 may include a semiconductor sensor that is provided specially for the bio-information detecting apparatus. Information from a car navigating system (not shown) installed in the car may be input into the bio-information detecting apparatus, and the acceleration may be calculated from velocity information obtained through the car navigating system. Though the acceleration sensor 130 is used as an example of the vehicle behavior sensor in the example, in addition to the sensor 130, a velocity sensor, an angular velocity sensor, a displacement sensor, a rotation sensor, etc., may be used as the vehicle behavior sensor. The vibration of the body surface associated with the heartbeats can be detected even with any of these vehicle behavior sensors and, thereby, the heartbeats can be detected similarly to the case when the installed-in-seat pressure sensor 110 or the acceleration sensor 130 is used.

The controller 120 includes a semiconductor memory, a CPU, etc., and also includes a memory unit 121, a calculating unit 122, a judging unit 123, a control unit 124, and an input setting unit 125. The detected signals obtained by the steering wheel sensor 100, the installed-in-seat pressure sensor 110, and the acceleration sensor 130 are input into the controller 120 to be processed. This controller 120 may be provided specially for the bio-information detecting apparatus or be configured to be in common with a control unit (not shown) of the car navigating system installed in the car.

The reporting device 140 is configured to output a guidance voice or buzzer sound based on a signal output from the control unit 124 of the controller 120. For example, in this example, when drowsiness of the driver has been detected by the detection using the steering wheel sensor 100 and the installed-in-seat pressure sensor 110, a control signal is output from the control unit 124 of the controller 120 to the reporting device 140 to attract attention of the driver. As a result, buzzer sound is output from a sound output unit (not shown) of the reporting device 140.

Otherwise, the reporting device may be configured to include a sound synthesizing unit (not shown) and synthesize, based on the control signal from the controller 120, a guidance voice such as "How about taking a break?" to output this guidance voice from the sound output unit (not shown). The reporting device 140 may be configured to be included in the car navigating system (not shown) installed in the car and guide the way to a nearest resting place by searching using the car navigating system.

The components of the bio-information detecting apparatus are not limited to those shown in Fig. 1 and other components may be included therein. For example, though not shown in Fig. 1, the bio-information detecting apparatus may be configured to further include an amplifier that amplifies the detected signal output from the steering wheel sensor 100, the installed-in-seat pressure sensor 110, and the acceleration sensor 130, an A/D converter that converts this detected signal from an analog signal to a digital signal, a filter that processes a signal etc., and to install these components between the sensors 100, 110, 130 and the controller 120.

A bio-information detecting method (more specifically, a drowsiness detecting method) according to this example using the bio-information detecting apparatus having the above configuration will be described below referring to Fig. 2. As shown in Fig. 2, in the bio-information detecting method in the example, an initial setting operation is executed prior to the main operation of detection when the driver has got in the car (in other words, at the start of driving). The main operation that is a detecting operation is executed based on the setting in the initial setting operation.

Usually, a driver can be regarded as having little drowsiness (in other words, be in an awaken state) when the driver gets in the car. Therefore, this awaken state of the driver is defined as a "reference state". In this reference state, the potential difference between the hands detected when each of the right and left hands grabs respectively the electrodes 100a and 100b (refer to Fig. 1) of the steering wheel sensor 100 (refer to Fig. 1) is obtained as an electric signal and is input into the controller 120 through the amplifier and the A/D converter (both not shown in Fig. 1) (step S1 shown in Fig. 2).

Because the driver sets out driving with seriousness when the driving is started, the driver often holds the steering wheel with both hands without noticing it. Therefore, the detection by this steering wheel sensor 100 is easily enabled.

In the controller 120, the calculating unit 122 (refer to Fig. 1) calculates the heart rate in the reference state using the electric signal input (step S2 shown in Fig. 2). The calculating unit 122 (refer to Fig. 1) calculates the heartbeat interval in the reference state using the electric signal input and the result of this calculation is stored in the memory unit 121 (step S3 shown in Fig. 2). The time interval of the heartbeats in the reference state obtained in this manner (hereinafter, "heartbeat-interval reference value") is used as a reference value for calculating the HF in the detecting operation described later.

The control unit 124 of the controller 120 sets a fluctuation range HR1 to HR2 of the heart rate based on the heart rate in the reference state calculated, and this fluctuation range HR1 to HR2 is stored in the memory unit 121 (refer to Fig. 1) (step S4 shown in Fig. 2). For example, when the heart rate in the reference state calculated is 70, the fluctuation range HR1 to HR2 is set in a range of ±10 from the heart rate 70. In other words, in this case, the fluctuation range HR1 to HR2 becomes 60 to 80.

The initial setting operation as above is executed by, for example, the driver by selecting the initial setting operation using the input setting unit 125 (refer to Fig. 1) when the driver has got in the car. When the initial setting operation has been selected, detection under the reference state is executed for the above initial setting for a predetermined period of time from the time when the driver has got in the car. Then, the fluctuation ranges HR1 to HR2 of the heartbeats and the heartbeat-interval reference value to be used in the detecting operation while driving are set. When these settings have been completed, the operation is shifted automatically to the detecting operation that is the main operation of the bio-information detecting apparatus, and the detecting operation is started.

The initial setting operation as above may be executed every time the driver gets in the car, or be executed regularly. When the driver has been changed, the heart rate and the heartbeat-interval reference value also change according to the driver. Therefore, in this case, the initial setting operation is executed again, or the initial setting values for each driver may be stored and the initial settings may be automatically changed by identifying a driver. Means of identifying a driver may be key input of IDs, etc., finger prints, voices, facial characteristics, and other bio-information.

The main operation of the drowsiness detection while driving will be described. The judging unit 123 (refer to Fig. 1) of the controller 120 (refer to Fig. 1) judges whether the signal can be detected from the steering wheel sensor 100 (refer to Fig. 1) (step S11 shown in Fig. 2). For example, when the driver holds the steering wheel with both hands and each of the right and left hands contacts respectively the electrodes 100a and 100b (refer to Fig. 1) of the steering wheel sensor 100 (refer to Fig. 1), the potential difference between the hands is detected by the electrodes 100a and 100b and an electric signal corresponding to this potential difference is output from the steering wheel sensor 100. Therefore, in this case, the judging unit 123 detects the output of the electric signal from the steering wheel sensor 100 and judges that detection from the steering wheel sensor 100 is possible (step S11: YES shown in Fig. 2).

When it is judged that the detection from the steering wheel sensor 100 (refer to Fig. 1) is possible, the electric signal output from the steering wheel sensor 100 is input into the calculating unit 122 (refer to Fig. 1) of the controller 120 (refer to Fig. 1) (step S13 shown in Fig. 2). The calculating unit 122 calculates the heart rate and the heartbeat time interval using this electric signal. The calculating unit 122 compares the calculated heartbeat time interval with the heartbeat-interval reference value stored in the memory unit 121 (refer to Fig. 1) by the above initial setting operation and calculates the fluctuated amount of the detected heartbeat time interval to the heartbeat-interval reference value, more specifically, the fluctuated amount of the HF (step S14 shown in Fig. 2).

The control unit 124 (refer to Fig. 1) of the controller 120 detect the drowsiness of the driver based on the heart rate and the HF obtained in this manner. More specifically, relative evaluation of the drowsiness is executed by detecting the degree of advancement of the drowsiness based on the obtained fluctuation of the heart rate, and absolute evaluation of the drowsiness is executed by detecting the presence or absence of the drowsiness or the degree of strength of the drowsiness based on the obtained HF (step S15 shown in Fig. 2). The details of each detection will be described below.

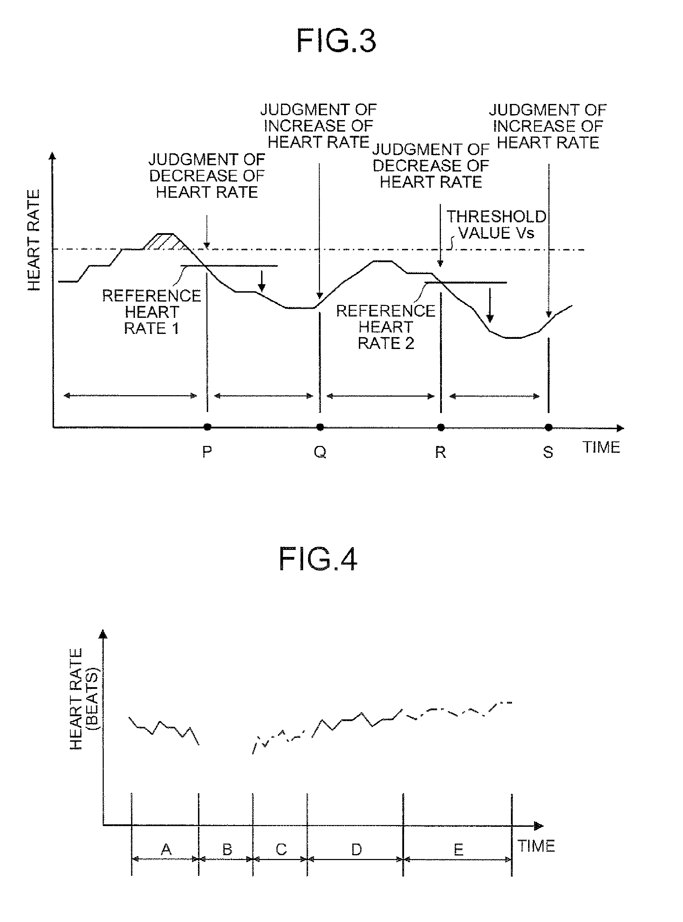

The detection of the degree of advancement of drowsiness based on the heart rate will be described. Fig. 3 is a schematic for explaining a detecting method of the degree of advancement of drowsiness based on the fluctuation of the heart rate. As shown in Fig. 3, the calculating unit 122 (refer to Fig. 1) calculates the amount of reduction of the heart rate based on the fluctuation of the detected heart rate, and the control unit 124 (refer to Fig. 1) detects the degree of advancement of drowsiness based on the calculated result.

More specifically, based on the fact that the amount of reduction of the heart rate increases as the drowsiness advances, the degree of advancement of drowsiness is estimated relatively from the fluctuation of the amount of reduction of the calculated heart rate. In this detection of the amount of reduction of the heart rate, a reference heart rate for calculating the amount of reduction of the heart rate is set and the amount of reduction to this reference heart rate is calculated. Taking into account the fact that the level of awakening of the driver always fluctuates, this reference heart rate is set in response to the fluctuation of the heart rate as appropriately every time reduction of the heart rate is judged.

For example, in this case, a detected heart rate at a time P at which the fluctuation of the heart rate has been judged to be decreasing is set as a reference heart rate 1, and the amount of reduction of the heart rate to the reference heart rate 1 is calculated. As a result of the calculation, the amount of reduction of the heart rate gradually increases during a time period from the time P to a time Q. Therefore, the drowsiness is estimated to be advancing. In this example, the amount of reduction of the heart rate is defined to be zero for the time period to the time P at which the judgment of reduction of the heart rate is executed.

Though the drowsiness advances during the time period from the time P to the time Q as described above, the driver becomes awaken again after the time Q and the heart rate increases to a time R. Therefore, the amount of reduction of the heart rate is zero from the time Q to the time R. When the fluctuation of the heart rate is judged to be decreasing again at the time R, this heart rate at the time R is set as a reference heart rate 2 and the amount of reduction of the heart rate to this reference heart rate 2 is calculated.

As a result of the calculation, the amount of reduction of the heart rate gradually increases during a time period from the time R to a time S. Therefore, the drowsiness is estimated to be advancing. In this case, because the amount of reduction of the heart rate during the time period from the time R to the time S is larger than the amount of reduction of the heart rate during the time period from the time P to the time Q, it can be relatively evaluated that the advancement of the drowsiness during the time period from the time R to the time S is larger than the advancement of the drowsiness during the time period from the time P to the time Q.

At this point, such an estimation of the drowsiness advancement based on only the amount of reduction of the heart rate judges even the amount of reduction of the heart rate caused by other reasons than the drowsiness as the advancement of the drowsiness. Therefore, false detection may be executed. More specifically, for example, when the driver becomes temporarily nervous because of overtaking another car, etc., and the heart rate of the driver is increased, the heart rate has been decreased (corresponding to the dashed portion in the figure) when the driver has returned to the original calm state. This decrease of the heart rate might be judged to be an indication of drowsiness.

Therefore, to prevent such wrong judgment, a threshold value Vs is set to the heart rate and the fluctuation of the heart rate equal to or less than this threshold value Vs is used to judge the drowsiness. The threshold value Vs is set as appropriate according to the detection precision, etc. and, for example, may be a value formed by adding 10 heartbeats to the heart rate in the reference state detected in the initial setting operation.

As a detecting method of the presence or absence of drowsiness and the degree thereof based on the HF, for example, the judging unit 123 (refer to Fig. 1) judges whether the increase rate for a predetermined time period (for example, 400 seconds) in the past is equal to or larger than a predetermined value (for example, 1.7 times as large as or larger) for the detected HF, and the judging unit 123 judges whether the fluctuation of the detected HF for a predetermined time period (for example, 100 seconds) in the past is increasing.

In this case, for example, the calculating unit 122 (refer to Fig. 1) calculates the slope of data of the HF detected for this predetermined time period (100 seconds) by applying a first-order regression equation thereto. When the calculated slope is positive, the judging unit 123 (refer to Fig. 1) judges that the HF is increasing, in other words, drowsiness is present. From the magnitude of the slope, the strength of the drowsiness is estimated. The above settings for time and increase rate in drowsiness detection based on the HF are made as appropriate based on experimental results.

As to the correlation between the increase of the HF and the occurrence of drowsiness, the correlation has been disclosed from the result of an experiment conducted separately to subjects. In this experiment, when the HF has increased, 65% of subjects responded that the drowsiness has strengthened considerably. Therefore, it has been confirmed that the HF can be used for the absolute evaluation of the presence or absence and the strength of drowsiness.

The case when the electro-cardio information from the steering wheel sensor 100 (refer to Fig. 1) can not be detected because the driver holds the steering wheel sensor 100 with one hand will be described. In this case, no potential difference between the hands is detected by the electrodes 100a and 100b of the steering wheel sensor 100. Therefore, no electric signal based on the potential difference is output from the steering wheel sensor 100 to the controller 120 (refer to Fig. 1). Therefore, the judging unit 123 (refer to Fig. 1) judges that the detection from the steering wheel sensor 100 is impossible (step S11: NO shown in Fig. 2).

In this case, instead of the detection by the steering wheel sensor 100 (refer to Fig. 1), executing the detection by the installed-in-seat pressure sensor 110 (refer to Fig. 1) can be considered. However, the installed-in-seat pressure sensor 110, due to the configuration thereof, detects the vibration of the body surface of the driver associated with the behavior of the vehicle (in other words, noise components) as heartbeats as described above. Therefore, high-precision detection can not be executed except the state where noise components are few, more specifically, a state where the car is running with few vibrations or the car is stopped. In this example, the judgment whether the detection by the installed-in-seat pressure sensor 110 is possible is executed as below.

The state where the detection by the installed-in-seat pressure sensor 110 is possible is, in other words, the state where the vehicle is running at an almost constant speed or is stopped. In these states, the fluctuation of the acceleration within a predetermined time period is almost zero.

Therefore, in this example, the calculating unit 122 (refer to Fig. 1) of the controller 120 (refer to Fig. 1) calculates the acceleration fluctuation for a predetermined time period based on acceleration information output from the acceleration sensor 130 (refer to Fig. 1). Based on this calculation result, the judging unit 123 (refer to Fig. 1) judges whether fluctuation of the acceleration within a predetermined time period is within a predetermined range and the acceleration is maintained within a predetermined range, in other words, whether the vehicle is stopped or running at a constant speed, and judges whether the detection by the installed-in-seat sensor 110 (refer to Fig. 1) is possible (step S12 shown in Fig. 2).

For example, when the range of the fluctuation of the acceleration within a predetermined time period is not within a predetermined range (in other words, when the fluctuation of the acceleration is large), the fluctuation of the speed of the vehicle is large and the car is not running stably. Therefore, vibration of the vehicle is large. Therefore, in this case, executing high-precision detection in the installed-in-seat pressure sensor 110 (refer to Fig. 1) is difficult. Therefore, in this case, the judging unit 123 (refer to Fig. 1) judges that detection by the installed-in-seat pressure sensor 110 is impossible (step S12: NO shown in Fig. 2) and the procedure is returned to step S11 shown in Fig. 2 again.

When the range of the fluctuation of the acceleration within a predetermined time period is within a predetermined range (in other words, when the fluctuation of the acceleration is small), the fluctuation of the speed of the vehicle is small and the car is running stably or is stopped. Therefore, the vibration of the vehicle is small. Therefore, in this case, executing high-precision detection by the installed-in-seat pressure sensor 110 (refer to Fig. 1) is possible. Therefore, in this case, the judging unit 123 (refer to Fig. 1) judges that detection by the installed-in-seat pressure sensor 110 is possible (step S12: YES of Fig. 2) and, following this, the detection by the installed-in-seat pressure sensor 110 is executed as below.

With the detection by the installed-in-seat pressure sensor 110, fluctuation of the pressing force to the sensor associated with the vibration of the body surface of the driver is input into the calculating unit 122 (refer to Fig. 1) of the controller 120 (refer to Fig. 1) as an electric signal (step S16 of Fig. 2) and, based on this electric signal, the calculating unit 122 calculates the heart rate (step S17 show in Fig. 2).

The judging unit 123 (refer to Fig. 1) judges whether the heart rate calculated as above is within a range [(HR1-ΔHR) to (HR2+ΔHR)] formed by considering a threshold ΔHR for the fluctuation range HR1 to HR2 of the heart rate that has been set in the initial setting operation and stored in the memory unit 121 (refer to Fig. 1) of the controller 120 (refer to Fig. 1) (step S18 shown in Fig. 2).

In other words, to enhance the detection precision of the installed-in-seat sensor 100 (refer to Fig. 1), referring to the fluctuation range HR1 to HR2 of the heart rate stored in the initial setting as above, the range of the normal detection of the heart rate by the installed-in-set pressure sensor 100 is defined as [(HR1-ΔHR) to (HR2+ΔHR)] using the threshold ΔHR. When the heart rate calculated at step S17 shown in Fig. 2 is within this range, the heart rate is considered as a normal value and, when the heart rate is another value, the heart rate is considered as a heart rate including noise components that are out of the normal value.

More specifically, when the heart rate calculated is not within the fluctuation rage (step S18: NO shown in Fig. 2), it is considered that the heart rate detected by the installed-in-seat pressure sensor 100 (refer to Fig. 1) includes many noise components such as vibrations associated with the behavior of the vehicle. Therefore, in this case, the judging unit 123 (refer to Fig. 1) judges that the detection by the installed-in-seat pressure sensor 100 is impossible, and the procedure is returned to step S11 shown in Fig. 2.

When the heart rate calculated is within the fluctuation rage (step S18: YES shown in Fig. 2), it is considered that the heart rate includes few noise components. Therefore, in this case, the judging unit 123 (refer to Fig. 1) judges that the detection by the installed-in-seat pressure sensor 100 is possible. Following this judgment, an acceleration component is subtracted from these detected signal components by the calculating unit 122 (refer to Fig. 1) (step S19 shown in Fig. 2) and the substantial heart rate is calculated because noise components other than the heartbeat information have been removed (step S20 shown in Fig. 2). Based on the substantial fluctuation of the heart rate calculated in this manner, the control unit 124 (refer to Fig. 1) detects the degree of advancement of the drowsiness in the method described in Fig. 3 (step S21 shown in Fig. 2).

As a method of removing the noise components at step S19 shown in Fig. 2, for example, the output signal from the installed-in-seat pressure sensor 100 (refer to Fig. 1) and the output signal from the acceleration sensor 130 may be converted respectively once by the control unit 124 (refer to Fig. 1) into frequency components, and the difference thereof may be calculated by the calculating unit 122 (refer to Fig. 1), and a process to re-convert into the original time-axis waveform may be applied again to the difference by the control unit 124.

In the detection by the installed-in-seat pressure sensor 110 (refer to Fig. 1), the heartbeats can not be detected directly as in the detection by the steering wheel sensor 100 (refer to Fig. 1). Therefore, the signal waveform output from the installed-in-seat pressure sensor 110 is a complicated waveform having many peaks compared to the signal waveform output from the steering wheel sensor 100. Therefore, in the detection by the installed-in-seat pressure sensor 110, detecting precisely the time interval of the heartbeats is difficult. Therefore, calculating the HF is not easy.

As described above, the result of the detection concerning the drowsiness detected from the steering wheel sensor 100 (refer to Fig. 1) and the installed-in-seat sensor 110 (refer to Fig. 1) is output from the control unit 124 (refer to Fig. 1) of the controller 120 (refer to Fig. 1) to the reporting device 140 (refer to Fig. 1) as a signal (step S22 shown in Fig. 2). For example, when the signal indicating that the drowsiness of the driver has been detected is input into the reporting device 140, the reporting device 140 makes buzzer sound based on this signal. Thus, the drowsiness of the driver is resolved. When the signal indicating that the driver is awake has been input into the reporting device 140, the reporting device 140 does not make the buzzer sound. In this manner, a reporting operation is executed in the reporting device 140 based on the output result from the controller 120 (step S23 shown in Fig. 2).

When a control signal to stop the detection operation has been input into the controller 120 (refer to Fig. 1) through the input setting unit 125 (refer to Fig. 1), or when the control signal to stop the detection operation has been automatically input into the controller 120 in accordance with the stop of the engine of the vehicle, the judging unit 123 (refer to Fig. 1) detects the input of this control signal (step S24: YES shown in Fig. 2). The control unit 124 (refer to Fig. 1) of the controller 120 stops the detection operation and the detection is ended. When the input of the control signal to stop has not been detected (step S24: NO shown in Fig. 2), the detection operation is continuously executed.

According to the above example, due to the apparatus configuration in which the steering wheel sensor 100 (refer to Fig. 1) and the installed-in-seat sensor 110 (refer to Fig. 1) are combined, the heartbeats can be detected by the steering wheel sensor 100 when the heartbeats can be detected by the steering wheel sensor 100 or by the installed-in-seat sensor 110 when the heartbeats can not be detected by the steering wheel sensor 100. Thus, the detection by the installed-in-seat pressure sensor 110 is possible though the driver does not notice the detection by the steering wheel sensor 100. Therefore, the detection can be executed easily without imposing any load on the driver.

Especially, in the detection by the installed-in-seat pressure sensor 110, the bio-information detecting apparatus judges, using the acceleration sensor 130, whether the detection by the installed-in-seat pressure sensor 110 is possible, and judges, utilizing the information detected from the steering wheel sensor 100 in the initial setting operation (more specifically, the fluctuation range HR1 to HR2 of the heart rate), whether the detection by the installed-in-seat sensor 110 is possible.

Therefore, even in the detection by the installed-in-seat pressure sensor 110 conventionally having lower detection precision, enhancement of the detection precision is facilitated and excellent detection can be executed. Thus, in the bio-information detecting apparatus according to the example, because the steering wheel sensor 100 and the installed-in-seat pressure sensor 110 are not simply combined, and the information detected by the steering wheel sensor 100 is utilized for the detection by the installed-in-seat pressure sensor 110, an effective advantage greater than that of the simple combination can be obtained.

Thus, the bio-information detecting apparatus according to the example can judge whether the situation is suitable for the operation of the steering wheel sensor 100 and the installed-in-seat pressure sensor 110 to select and use as appropriate the appropriate sensor that can detect according to the situation. And the bio-information detecting apparatus does not simply operate selectively the steering wheel sensor 100 and the installed-in-seat pressure sensor 110 that are combined according to the situation but can improve the detection performance of the installed-in-seat pressure sensor 110 by utilizing the detected information of the steering wheel sensor 100 having high detection precision and high reliability. Therefore, according to the bio-information detecting apparatus according to the example, stable and high-precision detection of drowsiness can be executed when the driver is not completely aware thereof.

The series of steps of the drowsiness detecting operation shown in Fig. 2 may be always executed consecutively while driving or may be executed at a predetermined time interval (for example, every 10 seconds).

### Second Embodiment

In a second embodiment, the detection of drowsiness by the bio-information detecting apparatus of the first embodiment described above will be described corresponding to the behavior of an actual vehicle. Fig. 4 is a schematic for explaining the operation of the bio-information detecting apparatus during various vehicle behaviors. Solid line portions of a graph shown in Fig. 4 (corresponding to a term A and a term D) indicate the result of the detection by the steering wheel sensor 100 (refer to Fig. 1) and chain line portions of the graph (corresponding to a term C and a term E) indicate the result of the detection by the installed-in-seat pressure sensor 110 (refer to Fig. 1). The portion showing neither a solid line nor a chain line (corresponding to a term B) indicates that no heart rate is detected.

For example, during the term "A" during which the vehicle is running straight on an ordinary road, the driver carefully drives concentrating his/her attention because the length of time passed from the start of the driving is short. Therefore, a state where the driver holds the steering wheel sensor 100 (refer to Fig. 1) with both hands is realized without being noticed by the driver. Therefore, in this case, high precision detection of the heartbeats can be executed by the steering wheel sensor 100.

On the other hand, during the term B, the steering wheel sensor 100 (refer to Fig. 1) can not be held with both hands due to the operation of winkers, etc., because the vehicle is running on an ordinary road having many curves and turnings to right and left at random speeds corresponding to the road conditions. Therefore, the detection by the steering wheel sensor 100 is impossible. In such a state, the detection by the installed-in-seat pressure sensor 110 (refer to Fig. 1) is also impossible. However, no drowsiness tends to occur because the driver especially concentrates his/her attention on his/her driving in such a situation. Therefore, it is not problematic that the detection operation is even stopped.

During the term C in which the vehicle is running on an ordinary road that is congested, the detection by the steering wheel sensor 100 is impossible because the steering wheel sensor 100 (refer to Fig. 1) is held with one hand. Therefore, the detection by the installed-in-seat pressure sensor 110 (refer to Fig. 1) is executed. In such a situation, a stable running state with little speed fluctuation (more specifically, a state close to a state where the vehicle is stopped) is realized and, therefore, this is a state suitable for the detection by the installed-in-seat pressure sensor 110. Therefore, high-precision and excellent detection influenced little by the noise components of the vibration associated with the behavior of the vehicle, etc., is realized by the installed-in-seat pressure sensor 110.

During the term D during which strong drowsiness occurs while running on a highway on which monotonous driving continues, the driver often holds the steering wheel sensor 100 (refer to Fig. 1) with both hands unconsciously to avoid danger. Therefore, the detection by the steering wheel sensor 100 is executed. In such a state where drowsiness has occurred, higher precision for detecting the drowsiness is required. However, as described above, detection of not only the heart rate but also the HF is possible in the detection by the steering wheel sensor 100 and the detection of drowsiness is executed using both of the relative evaluation and the absolute evaluation. Therefore, high-precision and excellent detection can be executed.

During the term E during which the driver drives with one hand on a highway, the detection by the steering wheel sensor 100 (refer to Fig. 1) is impossible and the detection by the installed-in-seat pressure sensor 110 (refer to Fig. 1) is executed. In such a situation, a state where the car is funning stably on a relatively flat and straight road (more specifically, a state where the car is running stably at a constant speed) is realized and this state is suitable for the detection by the installed-in-seat pressure sensor 110. Therefore, high-precision and excellent detection influenced little by the noise components of the vibration associated with the behavior of the vehicle, etc., is realized by the installed-in-seat pressure sensor 110.

As described above, according to the drowsiness detecting method of the example, in the various behaviors of the vehicle, an appropriate detecting method is selected as appropriate, and the excellent detection for which improvement of the precision has been facilitated can be executed. Therefore, the advantages of the bio-information detecting apparatus can be effectively exerted in any behavior of a vehicle. Therefore, this bio-information detecting apparatus can effectively prevent the driver from dozing off at the wheel.

### Third Embodiment

In the first embodiment described above, the case where whether the detection by the installed-in-seat pressure sensor (refer to Fig. 1) is possible is judged based on the fluctuation of the acceleration detected by the acceleration sensor 130 (refer to Fig. 1) and the range HR1 to HR2 of the fluctuation of the heartbeats set in the initial setting operation (refer to Fig. 2) is described. However, in a third embodiment of the present invention, this judgment is executed using a pressure-sensor output-template waveform created and stored in the initial setting operation as a parameter. The configuration of the bio-information detecting apparatus according to the example is same as the configuration of the apparatus shown in Fig. 1 described above and, therefore, the description thereof is omitted.

Describing the overview of the example more specifically, the detection of the heartbeats by the steering wheel sensor 100 (refer to Fig. 1) and the detection of the heartbeats by the installed-in-seat pressure sensor 110 (refer to Fig. 1) are simultaneously executed as the initial setting operation. The correlation between a steering-wheel-sensor output waveform and a pressure-sensor output waveform obtained in the detection is checked. As described above, the detection of the heartbeats can be executed directly in the detection by the steering wheel sensor 100. Therefore, when the correlation between the obtained pressure-sensor output waveform and the steering-wheel-sensor output waveform is strong, it is considered that a normal heartbeat signal is detected from the installed-in-seat pressure sensor 110. Therefore, the pressure-sensor output-waveform at this time is stored in the memory unit (refer to Fig. 1) of the controller 120 (refer to Fig. 1) as the pressure-sensor output-template waveform.

When the detection by the steering wheel sensor 100 (refer to Fig. 1) is impossible in the detection operation while driving, the correlation between the pressure-sensor output-waveform obtained from the installed-in-seat pressure sensor 110 (refer to Fig. 1) and the above pressure-sensor output-template waveform is checked, and whether the detection by the installed-in-seat pressure sensor 110 is possible is judged based on the degree of the correlation. Thus, the precision of the detection by the installed-in-seat pressure sensor 110 can be improved. The details of the above procedure will be described.

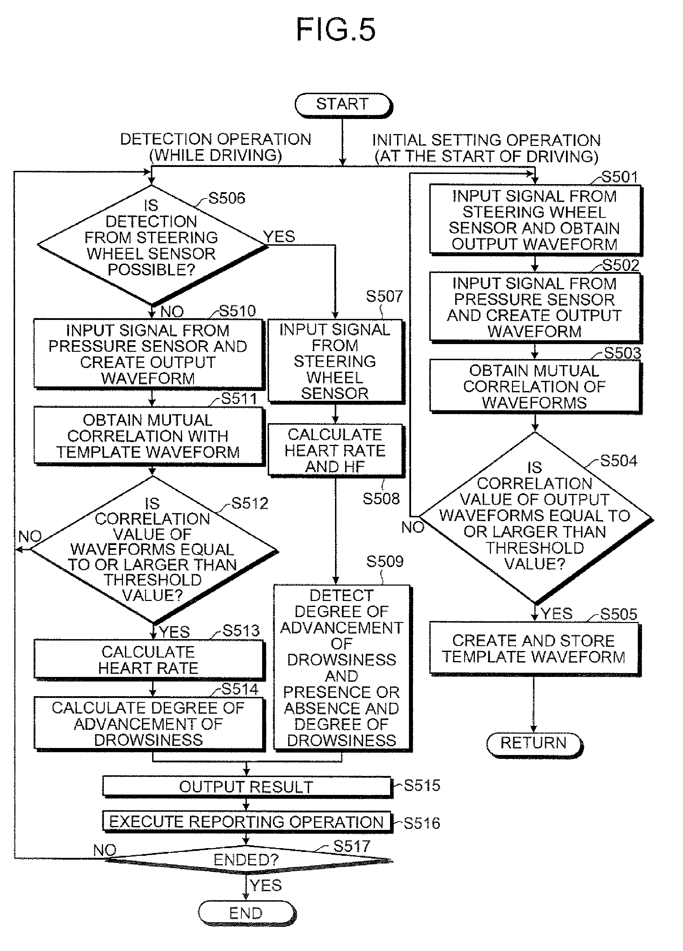

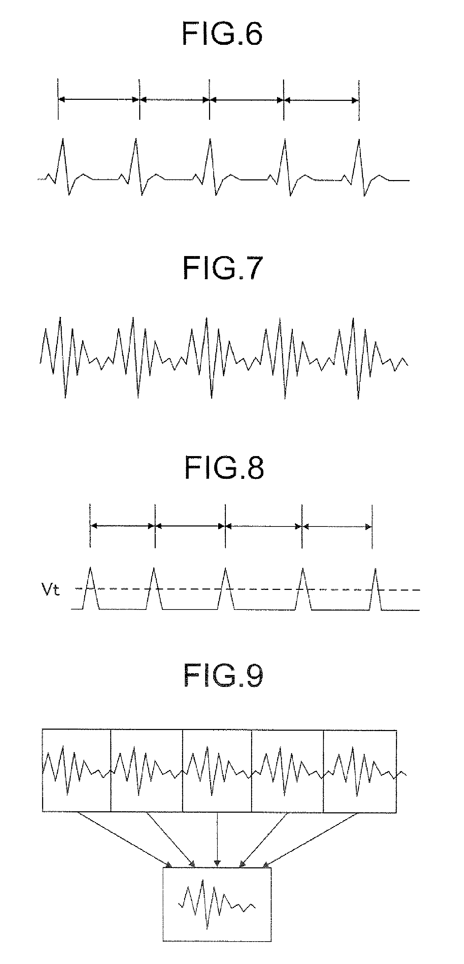

Fig. 5 is a flowchart of the drowsiness detecting method in the example. Figs. 6 to 9 are schematics for explaining a method of creating the pressure-sensor output-template waveform used in the drowsiness detecting method shown in Fig. 5. Fig. 6 is a schematic of a heartbeat waveform output from the steering wheel sensor 100 (refer to Fig. 1) in the initial setting operation for creating the template waveform (in other words, the steering-wheel-sensor output waveform). Fig. 7 is a schematic of a heartbeat waveform output from the pressure sensor (the pressure-sensor output waveform) simultaneously as the output by the steering wheel sensor 100 shown in Fig. 6.

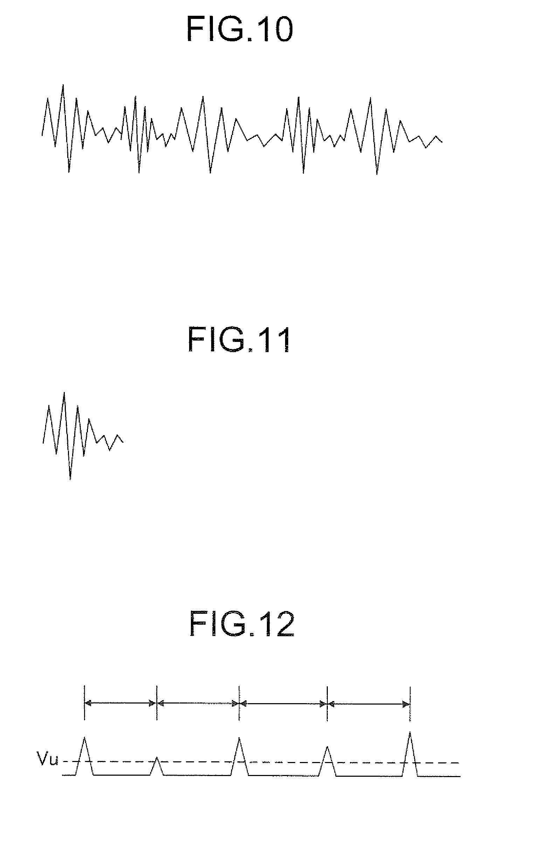

Fig. 8 is a schematic for illustrating the result of calculation of the mutual correlation between the steering-wheel-sensor output waveform shown in Fig. 6 and the pressure-sensor output waveform shown in Fig. 7. Fig. 9 is a schematic of a pressure-sensor output-template waveform created. Figs. 10 to 12 are explanatory views of a judging method of detection of the installed-in-seat pressure sensor descried later in the drowsiness detecting method shown Fig. 5.

According to the bio-information detecting method of the example, as shown in Fig. 5, in the initial setting operation, both of the steering wheel sensor 100 (refer to Fig. 19 and the installed-in-seat pressure sensor 110 (refer to Fig. 1) are simultaneously operated and detection of the heartbeats is executed by each of the sensors. A signal output from the steering wheel sensor 100 is input into the controller 120 (refer to Fig. 1) and, from this signal, the control unit 124 (refer to Fig. 1) creates the steering-wheel-sensor output waveform (refer to Fig. 6) (step S501 shown in Fig. 5). A signal output from the installed-in-seat pressure sensor 110 (refer to Fig. 1) is input into the controller 120 (refer to Fig. 1) and, from this signal, the control unit 124 (refer to Fig. 1) creates the pressure-sensor output waveform (refer to Fig. 7) (step S502 shown in Fig. 5).

Based on the steering-wheel-sensor output waveform (refer to Fig. 6) and the pressure-sensor output waveform (refer to Fig. 7) obtained as above, the calculating unit 122 (refer to Fig. 1) calculates the mutual correlation between these output waveforms according to a mutual correlation computing equation. As a result, a correlation value that indicates the mutual correlation between the steering-wheel-sensor output waveform and the pressure-sensor output waveform as shown in Fig. 8 is obtained (step S503 shown in Fig. 5). As shown in Fig. 8, for the result of the calculation of the mutual correlation, when the correlation value is equal to or larger than a threshold value Vt, and the average value and the dispersion (the standard deviation) of peak intervals of the correlation value are respectively close to the average value and the dispersion (the standard deviation) of peak intervals of the steering-wheel-sensor output waveform, it is judged that the correlation between the pressure-sensor output waveform and the steering-wheel sensor output waveform is strong (step S504: YES shown in Fig. 5). When the pressure-sensor output waveform having the strong correlation with the steering-wheel sensor output waveform has been obtained as above, as shown in Fig. 9, this pressure-sensor output waveform is stored in the memory unit (refer to Fig. 1) as the pressure-sensor output-template waveform (step S505 shown in Fig. 5).

The threshold value Vt (refer to Fig. 8) can be set arbitrarily and is set as appropriate considering the precision of the bio-information detection. When the calculated correlation value is smaller than the threshold value Vt (step S504: NO shown in Fig. 5), the correlation between the pressure-sensor output waveform (refer to Fig. 7) and the steering-wheel-sensor output waveform (refer to Fig. 6) is weak. Therefore, the pressure-sensor output waveform includes vibration components other than the heartbeats (in other words, noise components). Therefore, when this pressure-sensor output waveform is used in the detection operation as the pressure-sensor output-template waveform (refer to Fig. 9), this results in degradation of the detection precision. Therefore, in this case, the procedure is returned to step S501 shown in Fig. 5 again.

The creating method of the pressure-sensor output-template waveform after the correlation has been checked will be described in detail. The control unit 124 (refer to Fig. 1) of the controller 120 (refer to Fig. 1), as shown in Fig. 9, executes a process of cutting out the pressure-sensor output waveform for each cycle. The calculating unit 122 (refer to Fig. 1) calculates the average waveform for each cycle that has been cut out. The average waveform obtained in this manner is stored in the memory unit 121 (refer to Fig. 1) as the pressure-sensor output-template waveform.

In addition to creating the pressure-sensor output-template waveform by obtaining the average waveform from the waveforms of all the cycles that have been cut out as above, the pressure-sensor output-template waveform may be obtained by selecting a waveform of the cycle having the highest correlation value with the steering-wheel-sensor output waveform (refer to Fig. 6) from the waveforms of the cycles that have been cut out.

Detection operation using the pressure-sensor output-template waveform created as above will be described. As shown in Fig. 5, similarly to the case of the first embodiment shown in Fig. 2, the judging unit 123 (refer to Fig. 1) of the controller 120 (refer to Fig. 1) judges whether the signal detection from the steering wheel sensor 100 (refer to Fig. 1) is possible, in other words, whether the steering wheel sensor 100 is held by both hands (step S506 shown in Fig. 5). When the signal detection from the steering wheel sensor 100 is possible (step SS06: YES shown in Fig. 5), operations (step S507 to step S509 shown in Fig. 5) same as step S13 to step S15 in the first embodiment shown in Fig. 2 are executed.

On the other hand, when the signal detection from the steering wheel sensor 100 (refer to Fig. 1) is impossible (step S506: NO shown in Fig. 5), the detection by the installed-in-seat pressure sensor 110 (refer to Fig. 1) is executed and the detected signal thereof is input into the controller 120 (refer to Fig. 1). The control unit 124 (refer to Fig. 1), as shown in Fig. 10, creates the pressure-sensor output waveform based on this signal (step S510 shown in Fig. 5). The calculating unit 122 (refer to Fig. 1) calculates the mutual correlation between these output waveforms according to a mutual correlation computing equation from the pressure-sensor output waveform obtained as above and the pressure-sensor output-template waveform shown in Fig. 11 that has been created and stored in the initial setting operation described above. Thus, the calculation result of the mutual correlation shown in Fig. 12 is obtained (step S511 shown in Fig. 5).

From the result of the mutual correlation calculated as above, as shown in Fig. 12, the judging unit 123 (refer to Fig. 1) judges whether the correlation value between the pressure-sensor output waveform (refer to Fig. 10) obtained in the detection operation and the pressure-sensor output-template waveform (refer to Fig. 11) is equal to or larger than a threshold value Vu. This threshold value Vu is set as appropriate corresponding to the detection precision, similarly to the threshold value Vt shown in Fig. 8.

When the correlation value is equal to or larger than the threshold value Vu (step S512: YES shown in Fig. 5), the correlation between the pressure-sensor output waveform (refer to Fig. 10) obtained in the detection operation and the pressure-sensor output-template waveform (refer to Fig. 11) is strong and, therefore, the correlation between the pressure-sensor output waveform obtained and the steering-wheel-sensor output waveform (refer to Fig. 6) may be regarded strong. Therefore, such improvement of precision as made in the detection by the steering wheel sensor 100 is facilitated in the detection by the installed-in-seat pressure sensor 110 (refer to Fig.
1). Therefore, in this case, the calculating unit 122 (refer to Fig. 1) calculates the heart rate using the output signal from the installed-in-seat pressure sensor 110 (step S513 shown in Fig. 5) and the control unit 124 (shown in Fig. 1) detects the degree of advancement of the drowsiness based on the obtained fluctuation of the heart rate (step S514 shown in Fig. 5).

On the other hand, when the correlation value is less than the threshold value Vu (step S512: NO shown in Fig. 5), the correlation between the pressure-sensor output waveform (refer to Fig. 10) obtained in the detection operation and the pressure-sensor output-template waveform (refer to Fig. 11) is weak and, therefore, the correlation between the pressure-sensor output-waveform obtained and the steering-wheel-sensor output waveform (refer to Fig. 6) may be regarded weak. Therefore, in such detection by the installed-in-seat pressure sensor 110 (refer to Fig. 1), the detected signal includes the noise components other than the vibration of the surface of the body caused by the heartbeats and, therefore, excellent detection can not be executed. Therefore, in this case, detection operation is executed again returning to step S506.

Steps (steps S515 to S517 shown in Fig. 5) after the detection of the degree of advancement, presence or absence, and the degree of drowsiness by the steering wheel sensor 100 (refer to Fig. 1) (step S509 shown in Fig. 5) or the detection of the degree of advancement of drowsiness by the installed-in-seat pressure sensor 110 (refer to Fig. 1) (step S514 shown in Fig. 5) are same as steps S22 to S24 in the first embodiment shown in Fig. 2 and, therefore, the description thereof is omitted.

As described above, also in the example having the above configuration, the same effect as that of the first embodiment can be exerted. In the example, obtaining the peak interval in a mutual correlation waveform shown in Fig. 12 obtained by calculating the mutual correlation between the pressure-sensor output waveform (refer to Fig. 10) and the pressure-sensor output-template waveform (refer to Fig. 11) is equivalent to obtaining the heartbeat interval of the heartbeats. Therefore, not only the heart rate but also the HF can be detected in the detection by the installed-in-seat pressure sensor 110 (refer to Fig. 1) and the absolute evaluation of drowsiness can be executed by detecting the presence or absence and the degree of drowsiness.

### Fourth Embodiment

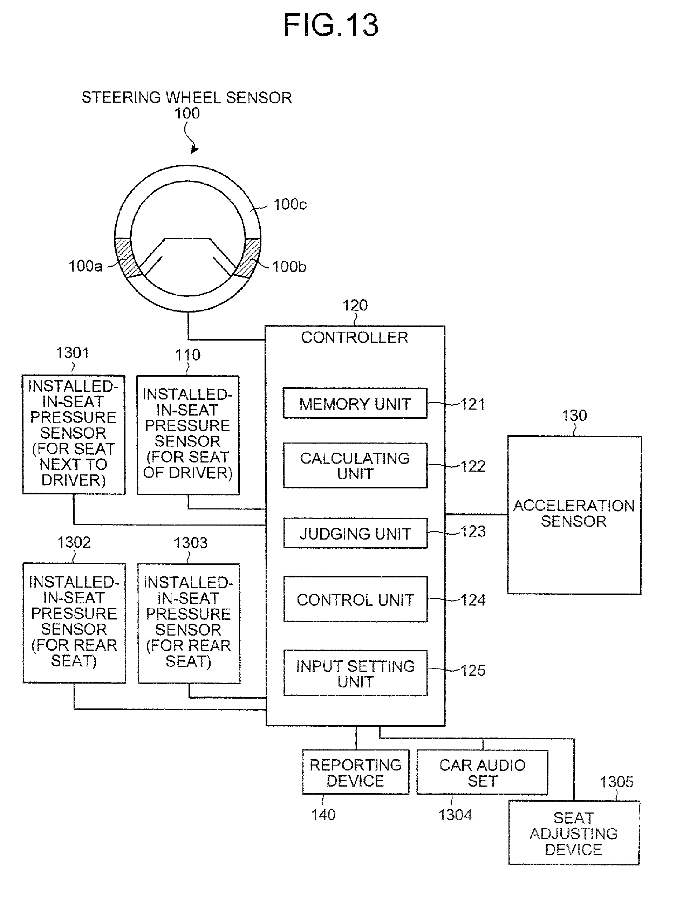

Fig. 13 is a schematic of the configuration of a bio-information detecting apparatus according to a fourth embodiment of the present invention. As shown in Fig. 13, the bio-information detecting apparatus of the example has the same configuration as that of the bio-information detecting apparatus of the first embodiment shown in Fig. 1. However, the present apparatus differs in the following points from that of the first embodiment. In other words, in the example, in addition to the seat of the driver, installed-in-seat pressure sensors 1301 to 1303 are respectively installed in the seat next to the seat of the driver and two rear seats of the vehicle. Signals detected by these installed-in-seat pressure sensors 1301 to 1303 are configured to be able to be output to the controller 120. In the example, detection of drowsiness of an accompanying passengers in the seat next to the seat of the driver and the rear seats (all not shown) is executed based on the signals detected by these installed-in-seat pressure sensors 1301 to 1303 and, in response to the result of the detection, the controller 120 is configured to control a car audio set 1304 and a seat adjusting apparatus 1305.

According to the bio-information detecting apparatus of the example, detection of drowsiness of the driver is executed according to the method of the first embodiment shown in Fig. 2 or the method of the third embodiment shown in Fig. 5. Estimation of the mental state (more specifically, detection of drowsiness) of the accompanying passengers is executed using the installed-in-seat pressure sensors 1301 to 1303 according to the following method.

In other words, in the example, for example, the detection of the degree of advancement of drowsiness of the accompanying passengers is executed through steps same as steps S12 to S21 in the first embodiment shown in Fig. 2. In such detection, similarly to the case of step S18 of the first embodiment, the detected information of the heartbeats of the driver by the steering wheel sensor 100 (refer to Fig. 1) (more specifically, heartbeat fluctuation range HR1 to HR2) is utilized in judging whether detection of heartbeats of the accompanying passengers by the installed-in-seat pressure sensors 1301 to 1303 is possible. Therefore, improvement of the precision of the heartbeat detection of the accompanying passengers by the installed-in-seat pressure sensors 1301 to 1303 can be facilitated.

Otherwise, in the example, the detection of the degree of advancement of drowsiness of the accompanying passengers may be executed through steps same as step S510 to step S514 in the third embodiment shown in Fig. 5. In this case, similarly to the case of steps S510 to S512, as shown in Figs. 10 to 12, the calculating unit 122 of the controller 120 (refer to Fig. 13) calculates the correlation between each pressure-sensor output waveform (refer to Fig. 10) created based on each signal detected by the installed-in-seat pressure sensors 1301 to 1303, and the pressure-sensor output-template waveform (refer to Fig. 11) created based on the heartbeats of the driver.

Based on the result of this calculation (refer to Fig. 12), whether detection of heartbeats of the accompanying passengers by the installed-in-seat pressure sensors 1301 to 1303 is possible is judged. Thus, improvement of the precision of the heartbeat detection of the accompanying passengers can be facilitated compared to the case where detection is executed by causing the installed-in-seat pressure sensors 1301 to 1303 to respectively operate individually.

When drowsiness or drowsing of the accompanying passengers is detected in the above method, the control unit 124 of the controller 120 automatically operates and controls the car audio set 1304 and the seat adjusting apparatus 1305 not to interfere the state of the accompanying passengers. More specifically, for example, the control unit 124 may switch the directivity of speakers (not shown) of the car audio set 1304 to let only the driver hear the sound; may automatically select songs based on the taste of the accompanying passengers other than those accompanying passengers of whom drowsiness has been detected, and may play the songs; or may incline seats of the accompanying passengers of whom drowsiness has been detected.

As described above, according to the example of the above configuration, the effects described in the first embodiment can be exerted, and the result of the detection of the mental state of the accompanying passengers can be applied to the car audio set 1304 and the seat adjusting apparatus 1305. Therefore, a comfortable state can be realized.

### (Other Variations)

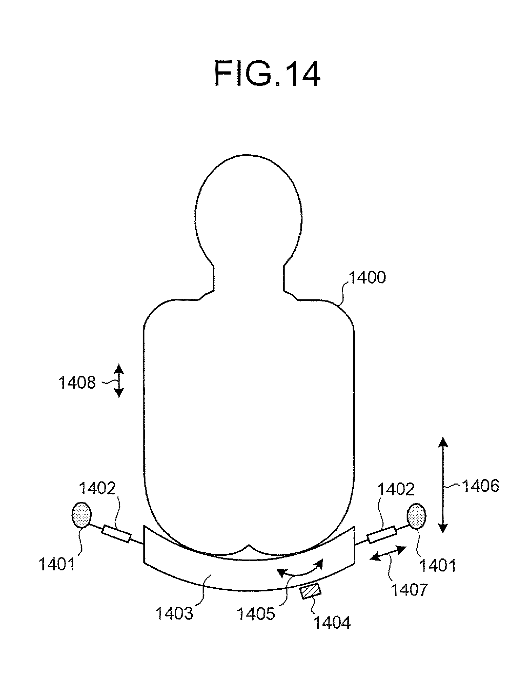

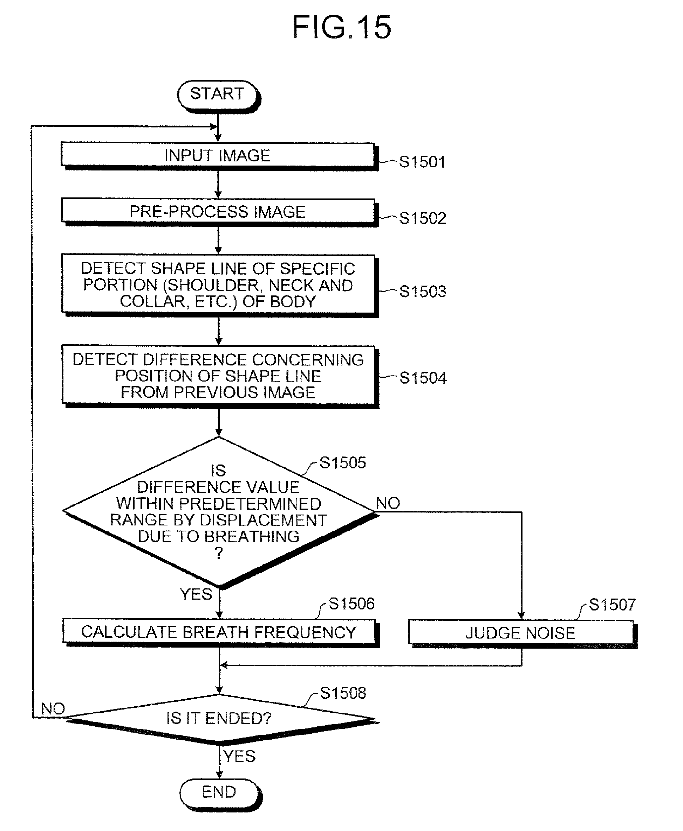

As other variations, it can be considered to apply an angular velocity sensor or a rotation sensor instead of the installed-in-seat pressure sensor 110. An image sensor can be used instead of the installed-in-seat pressure sensor 110. These configurations will be described referring to Fig. 14 and Fig. 15.

Fig. 14 is a schematic of the configuration of a seat in the case when an angular velocity sensor or a rotation sensor is applied. A seating face 1403 is attached to a frame 1401 of a chair through an elastic body 1402. An angular velocity sensor or a rotation sensor 1404 is fixed to the seating face 1403. A passenger 1400 sits on the seating face 1403. The angular velocity sensor or the rotation sensor 1404 plays the role of the installed-in-seat pressure sensor 110.

The angular velocity sensor or the rotation sensor 1404 attached to the seating face 1403 cancels the vertical vibration of the chair and detects a fine rotation 1405 of the seating face 1403 caused by a heartbeat vibration. Vibrations 1406 of the car being running is transmitted from the frame 1401 to the elastic body 1402. However, the elastic body 1402 absorbs most of the vibrations by the telescopic motion thereof in a longitudinal direction 1407. On the other hand, the vibration caused by the heartbeats is transmitted to the seat as a vibration 1408 of the blood flowing through the arteries. The angular velocity sensor or the rotation sensor 1404 detects the fine rotation 1405 of the seating face 1403 caused by the heartbeat vibration as heartbeat information and inputs the information into the controller 120. The processing of the heartbeat information is as described in the first embodiment.

Fig. 15 is a flowchart of the process of calculating a breath frequency using a pair of electrodes in the steering wheel portion. The breath frequency can be extracted from the fluctuation component of the heartbeats. A heart beats due to the adjustment balance of the autonomic nervous system. A fluctuating component of the heartbeat time interval is "heartbeat fluctuation". A specific frequency component (=0.15 to 0.4 Hz) of this fluctuation is called "HF" and is influenced by breathing.

An image sensor is used instead of the installed-in-seat pressure sensor 110. By using this image sensor, the controller 120 shown in Fig. 1 calculates the breath frequency and, from the breath frequency, detects that drowsiness has occurred. First, an image is input (step S1501). In other words, a moving image of the upper body of the driver is shot by a camera attached to the seat of the driver. The image is pre-processed (step S1502). A shape line of a specific portion (a shoulder, the neck and collars, etc.) of the body is detected from the image (step S1503). The difference concerning the positions of the shape lines between a current image and the previous image is detected (step S1504). Thus, the difference of the position of the shape line of a specific portion (a shoulder, the neck and collars, etc.) of the body is regularly detected.

Whether the value of the difference is a value within a predetermined range by the displacement due to breathing is judged (step S1505). When the value is within the predetermined range (step S1505: YES), the breath frequency is calculated (step S1506). When the value is not within the predetermined range (step S1505: NO), the motion is not breathing and, therefore, is judged to be a noise (step S1507).

After the calculation of the breath frequency or judgment of the noise, whether the process has been finished is judged (step S1508). When the process has been finished (step S1508: YES), a series of steps is finished. When the process has not been finished (step S1508: NO), the procedure is returned to step S1501.

With the above process, a specific breath frequency component (=0.15 to 0.4 Hz) is extracted from the motion of the body. The amount of fluctuation of this specific breath frequency component is regarded as the breath rate. By using this breath rate, it can be judged whether drowsiness has occurred. For example, it can be judged that drowsiness has occurred when the breath rate is smaller than a predetermined rate.

The configuration of the bio-information detecting apparatus of the present invention is not limited to those of the first embodiment to fourth embodiment and may take other configurations. For example, a cardiac sound sensor may be installed in the seat instead of the installed-in-seat pressure sensor 110 (refer to Fig. 1). In this case, for example, as the cardiac sound sensor, a sensor available for a band expanded down to a frequency lower than that an ordinary microphone can detect (around 1 Hz) is utilized, and is installed in a portion of the seat corresponding to a portion under the buttocks, under the thighs, or on the back of the driver when the driver sits on the seat. Thus, the beats of the heart can be detected through the cardiac sound sensor as sound. Instead of the installed-in-seat pressure sensor 110, for example, a cardiac sound sensor or an acceleration sensor is installed in the seat belt portion, and the fluctuation of the cardiac sound or the acceleration synchronized with the beats of the heart may be detected.

Otherwise, the apparatus may be configured to add electrode pads to locations to which a hand that has left the steering wheel sensor 100 (refer to Fig. 1) is spontaneously put, for example, the parking brake bar, such that the potential difference can be detected between the hand and the other hand left on the steering wheel sensor 100. The apparatus may be configured to include contents to prevent drowsing after detecting the drowsiness, that stimulate the driver to notice his/her drowsiness by showing the driver the degree of advancement of the drowsiness as data by displaying the advancement of the drowsiness on a monitor in a graph, etc. The apparatus may be configured to output a message, etc., that stimulates the driver to take a break when necessary, or be configured to provide music, video images, or another information to resolve actively the drowsiness.

The above technique to estimate the state of the driver that utilizes the bio-information can be used not only for detecting and preventing drowsiness but also as a new interface between humans and cars, for example, medical uses, and recording means (driving recorder) to be used in an accident, etc. The technique can be used combining with a car audio system and a car navigating system that provides music and video images.

In the above description, a case when the bio-information detecting apparatus according to the present invention is applied to a car has been described. However, the apparatus can be applied to a vehicle other than a car, a ship, an airplane, etc.

## Claims

1. A bio-information detecting apparatus that is installed in a vehicle, a ship, or an airplane to be steered and detects at least mental and physical states of a driver, comprising:
an electrode sensor that includes at least a pair of electrodes and detects heartbeats by detecting potential difference between portions of a body that directly contact the electrodes; and
an installed-in-seat sensor that detects heartbeats by detecting vibration of a surface of the body associated with the heartbeats, wherein
the bio-information detecting apparatus is configured to be able to utilize heartbeat information obtained by detection by the electrode sensor for detecting heartbeats by the installed-in-seat sensor, and to judge whether detection by the installed-in-seat sensor can be executed based on the heartbeat information obtained by the electrode sensor when heartbeat detection by the electrode sensor can not be executed.

2. The bio-information detecting apparatus according to claim 1, wherein whether the detection by the installed-in-seat sensor can be executed is judged based on the heartbeat information obtained by the electrode sensor when the heartbeat detection by the electrode sensor can not be executed and, only when it is judged that the detection can be executed, heartbeat detection by the installed-in-seat sensor is executed.

3. The bio-information detecting apparatus according to claim 1, wherein the heartbeat information to be used for the heartbeat detection by the installed-in-seat sensor is a normal fluctuation range of heartbeats set based on normal heartbeats detected by the electrode sensor.

4. The bio-information detecting apparatus according to claim 3, wherein
a detectable-heartbeat fluctuation range that enables the heartbeat detection by the installed-in-seat sensor is set based on the normal fluctuation range of the heartbeats obtained by the electrode sensor, and
it is judged that the detection by the installed-in-seat sensor can be executed when heartbeats detected by the installed-in-seat sensor is within the detectable-heartbeat fluctuation range.

5. The bio-information detecting apparatus according to claim 1, wherein
the heartbeat detection by the electrode sensor and the heartbeat detection by the installed-in-seat sensor are simultaneously executed to calculate mutual correlation between an electrode-sensor output waveform obtained by the detection by the electrode sensor and an installed-in-seat-sensor output waveform obtained by the detection by the installed-in-seat sensor, and an installed-in-seat-sensor output-template waveform is formed based on a result of the calculation, and
when heartbeats are detected by the installed-in-seat sensor, the mutual correlation between an installed-in-seat-sensor output waveform obtained by the detection by the installed-in-seat sensor and the installed-in-seat-sensor output-template waveform is calculated, and whether the detection by the installed-in-seat sensor can be executed is judged based on a result of the calculation.

6. The bio-information detecting apparatus according to claim 5, wherein
when heartbeats are detected by the installed-in-seat sensor, the mutual correlation between the installed-in-seat-sensor output waveform obtained by the detection by the installed-in-seat sensor and the installed-in-seat-sensor output-template waveform is calculated, and whether the detection by the installed-in-seat sensor can be executed is judged based on a result of the calculation, and only when it is judged that the detection can be executed, the detection of heartbeats by the installed-in-seat sensor is executed.

7. The bio-information detecting apparatus according to claim 5, wherein
when correlation value between the installed-in-seat-sensor output waveform obtained by the detection by the installed-in-seat sensor and the installed-in-seat-sensor output-template waveform is equal to or larger than a predetermined value, it is judged that the detection by the installed-in-seat sensor can be executed.

8. The bio-information detecting apparatus according to any one of claims to 7, wherein
the bio-information detecting apparatus includes a target-to-drive behavior sensor that detects behavior of a target to drive, and is configured to be able to utilize behavior information of the target to drive obtained by detection by the target-to-drive behavior sensor during the heartbeat detection by the installed-in-seat sensor,
when the heartbeat detection by the electrode sensor can not be executed, whether the detection by the installed-in-seat sensor can be executed is judged based on the behavior information of the target to drive obtained by the target-to-drive behavior sensor.

9. The bio-information detecting apparatus according to claim 8, wherein
the target-to-drive behavior sensor detects acceleration of the target to drive and, when a fluctuation of the acceleration for a unit time period is within a predetermined range, it is judged that the detection by the installed-in-seat sensor can be executed.

10. The bio-information detecting apparatus according to any one of claims 1 to 7, wherein
the electrode sensor includes a pair of electrodes respectively installed separately on a steering wheel installed at a seat of the driver,
the installed-in-seat sensor is installed in a portion of the seat corresponding to a portion under buttocks, under thighs, or on back of the seat.

11. The bio-information detecting apparatus according to any one of claims 1 to 7, wherein
the installed-in-seat sensor is further installed in a portion of seats other than the seat of the driver, corresponding to a portion under the buttocks, under the thighs, or on the back, and the bio-information detecting apparatus is configured to be able to detect heartbeats of accompanying passengers sitting on the seats, and to be able to use heartbeat information of the driver obtained by the detection by the electrode sensor disposed on the steering wheel of the seat of the driver when heartbeats of the accompanying passengers are detected by the installed-in-seat sensor, and
whether heartbeat detection of the accompanying passengers by the installed-in-seat sensor can be executed is judged based on the heartbeat information of the driver obtained by the electrode sensor.

12. The bio-information detecting apparatus according to claims 11, wherein whether the heartbeat detection of the accompanying passengers by the installed-in-seat sensor can be executed is judged based on the heartbeat information of the driver obtained by the electrode sensor and, only when the detection can be executed, the heartbeat detection by the installed-in-seat sensor is executed.

13. The bio-information detecting apparatus according to any one of claims 1 to 7, wherein further comprising a reporting unit that reports bio-information obtained by the detection by the electrode sensor and the installed-in-seat sensor to at least the driver.

14. The bio-information detecting apparatus according to any one of claims 1 to 7, wherein the installed-in-seat sensor is any one of a pressure sensor, a displacement sensor, an acceleration sensor, an angular velocity sensor, and a rotation sensor.

15. A bio-information detecting apparatus that is installed in a vehicle, a ship, or an airplane to be steered and detects at least mental and physical states of a driver, comprising:
an electrode sensor that includes at least a pair of electrodes and detects a breath rate by detecting the potential difference between portions of a body that directly contact the electrodes; and
an installed-in-seat sensor or an image sensor that detects the breath rate by detecting vibration of a surface of the body associated with breathing, wherein
the bio-information detecting apparatus is configured to be able to utilize breath rate information obtained by detection by the electrode sensor for detecting a breath rate by the installed-in-seat sensor or the image sensor, and to judge whether detection by the installed-in-seat sensor or the image sensor can be executed based on the breath rate information obtained by the electrode sensor when breath rate detection by the electrode sensor can not be executed.

16. The bio-information detecting apparatus according to claim 15, wherein the installed-in-seat sensor is any one of a pressure sensor, a displacement sensor, an acceleration sensor, an angular velocity sensor, or a rotation sensor.
